(19) Europäisches Patentamt
European Patent Office
Office européen des brevets

(11) Veröffentlichungsnummer: **0 167 121 B1**

(12) **EUROPÄISCHE PATENTSCHRIFT**

(45) Veröffentlichungstag der Patentschrift: **23.10.91**

(51) Int. Cl.[5]: **C07D 213/85**, A61K 31/495

(21) Anmeldenummer: **85108004.4**

(22) Anmeldetag: **28.06.85**

Die Akte enthält technische Angaben, die nach dem Eingang der Anmeldung eingereicht wurden und die nicht in dieser Patentschrift enthalten sind.

# (54) Neue substituierte Phenylpiperazinyl-propanole, Verfahren zu ihrer Herstellung und ihre Vewendung sowei diese Verbindungen enthaltende Zubereitungen.

(30) Priorität: **05.07.84 DE 3424685**

(43) Veröffentlichungstag der Anmeldung:
**08.01.86 Patentblatt 86/02**

(45) Bekanntmachung des Hinweises auf die Patenterteilung:
**23.10.91 Patentblatt 91/43**

(84) Benannte Vertragsstaaten:
**AT BE CH DE FR GB IT LI NL SE**

(56) Entgegenhaltungen:
**EP-A- 0 005 828**
**EP-A- 0 008 645**

(73) Patentinhaber: **Beiersdorf Aktiengesellschaft**
**Unnastrasse 48**
**W-2000 Hamburg 20(DE)**

(72) Erfinder: **Stenzel, Wolfgang, Dr.**
**Lerchenweg 8**
**W-2057 Reinbek(DE)**
Erfinder: **Hofferber, Eva, Dr.**
**Emilienstrasse 47**
**W-2000 Hamburg 20(DE)**

**EP 0 167 121 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind neue substituierte Phenylpiperazinyl-propanole der Formel I,

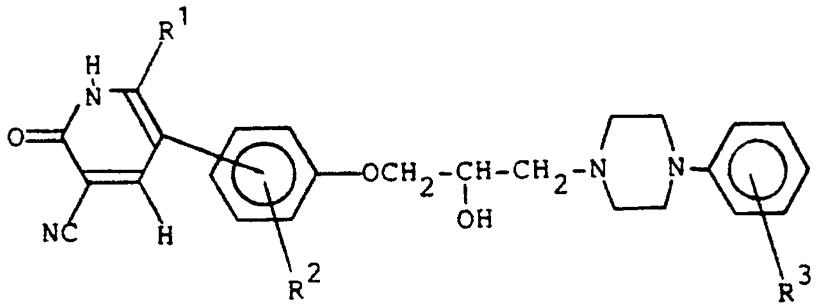

(I)

in der

$R^1$ Wasserstoff, eine Alkylgruppe mit 1 bis 4 Kohlenstoffatomen und

$R^2$ und $R^3$, die gleich oder verschieden sein können, jeweils Wasserstoff, Halogen, Cyano, eine Trifluormethylgruppe, eine Nitrogruppe, eine Hydroxygruppe, eine Alkoxygruppe oder eine Alkylgruppe mit jeweils 1 bis 4 Kohlenstoffatomen bedeuten, wobei die Alkylteile jeweils geradkettig oder verzweigt sein können, sowie ihre tautomeren Formen und ihre Salze sowie Säureadditionssalze und N-Oxide, Verfahren zu ihrer Herstellung und ihre Verwendung sowie diese Verbindungen enthaltende Zubereitungen.

Der Einfachheit halber sind die erfindungsgemäßen Verbindungen in nur einer durch Formel I wiedergegebenen tautomeren Form definiert. Die Erfindung erstreckt sich jedoch auf alle tautomeren Formen der Verbindungen.

Obgleich pharmazeutisch verträgliche Salze und Säureadditionssalze der neuen Verbindungen der Formel I und deren tautomere Formen und N-Oxide bevorzugt sind, liegen alle Salze innerhalb des Bereichs der Erfindung. Alle Salze sind wertvoll zur Herstellung der Verbindungen, selbst wenn das spezielle Salz nur als Zwischenprodukt gewünscht wird, wie zum Beispiel, wenn das Salz nur für Zwecke der Reinigung oder Identifizierung gebildet wird, oder wenn es als ein Zwischenprodukt bei der Herstellung eines pharmazeutisch verträglichen Salzes, beispielsweise durch Ionenaustauschverfahrensweisen, verwendet wird.

Die Verbindungen der allgemeinen Formel I und deren Salze enthalten asymmetrische Kohlenstoffatome. Daher sind auch die verschiedenen optischen Isomeren sowie die Diastereoisomeren Gegenstand der Erfindung ebenso wie die Salze und Additionssalze dieser Verbindungen mit Säuren. Die Racemate können nach an sich bekannten Methoden in ihre optischen Antipoden aufgetrennt werden.

Die Erfindung bezieht sich auch auf die N-Oxide der Verbindungen der allgemeinen Formel I. Sie sind nach bekannten Herstellungsverfahren erhältlich (H.S. MOSHER et al., Org. Synth., Coll. Vol. IV, 828, 1963).

Besonders bevorzugte Alkylgruppen sind die Methylgruppe und die Ethylgruppe. Halogen ist vorzugsweise Fluor oder Chlor. Bevorzugte Alkoxygruppen sind Methoxy- und Ethoxygruppen.

Der Pyridinonrest ist vorzugsweise in 4-Stellung am Phenylkern angeordnet.

Die folgenden Verbindungen der allgemeinen Formel I, deren N-Oxide und deren Salze werden bevorzugt:

1-[4-(3-Cyano-1,2-dihydro-2-oxo-pyridinyl-5)-phenoxy]-3-[4-(2-methoxyphenyl)-piperazinyl-1]-propanol-2

1-[3-(3-Cyano-1,2-dihydro-6-methyl-2-oxo-pyridinyl-5)-phenoxy]-3-[4-(2-methoxyphenyl)-piperazinyl-1]-propanol-2

1-[4-(3-Cyano-1,2-dihydro-6-methyl-2-oxo-pyridinyl-5)-phenoxy]-3-[4-methylphenyl)-piperazinyl-1]-propanol-2

1-[4-(3-Cyano-1,2-dihydro-6-methyl-2-oxo-pyridinyl-5)-2-methoxyphenoxy]-3-[4-(2-methoxyphenyl)-piperazinyl-1]-propanol-2

1-[4-(3-Cyano-1,2-dihydro-6-methyl-2-oxo-pyridinyl-5)-phenoxy]-3-[4-(3-methoxyphenyl)-piperazinyl-1]-propanol-2

1-[4-(3-Cyano-1,2-dihydro-6-methyl-2-oxo-pyridinyl-5)-2-methoxyphenoxy]-3-[4-(2-methylphenyl)-piperazinyl-1]-propanol-2

1-[4-(3-Cyano-1,2-dihydro-6-methyl-2-oxo-pyridinyl-5)-phenoxy]-3-(4-phenylpiperazinyl-1)-propanol-2

1-[3-(3-Cyano-1,2-dihydro-6-methyl-2-oxo-pyridinyl-5)-phenoxy]-3-(4-phenylpiperazinyl-1)-propanol-2-hydrochlorid
1-[2-(3-Cyano-1,2-dihydro-6-methyl-2-oxo-pyridinyl-5)-phenoxy]-3-(4-phenylpiperazinyl-1)-propanol-2
1-[4-(3-Cyano-1,2-dihydro-6-methyl-2-oxo-pyridinyl-5)-phenoxy]-3-[4-(4-hydroxyphenyl)-piperazinyl-1]-propanol-2
1-[4-(3-Cyano-1,2-dihydro-6-ethyl-2-oxo-pyridinyl-5)-phenoxy]-3-[4-(2-methoxyphenyl)-piperazinyl-1]-propanol-2

Besonders bevorzugt werden die folgenden Verbindungen der allgemeinen Formel I, deren N-Oxide und deren Salze mit einem hohen therapeutischen Wert, und zwar in der Form der Racemate sowie in der Form optisch aktiver Isomerer:
1-[4-(3-Cyano-1,2-dihydro-6-methyl-2-oxo-pyridinyl-5)-phenoxy)-3-[4-(2-methoxyphenyl)-piperazinyl-1]-propanol-2
1-[4-(3-Cyano-1,2-dihydro-6-methyl-2-oxo-pyridinyl-5)-phenoxy]-3-[4-(2-methylphenyl)-piperazinyl-1]-propanol-2
1-[4-(3-Cyano-1,2-dihydro-6-methyl-2-oxo-pyridinyl-5)-2-methoxyphenoxy]-3-[4-(2-methoxyphenyl)-piperazinyl-1]-propanol-2
1-[4-(3-Cyano-1,2-dihydro-6-methyl-2-oxo-pyridinyl-5)-phenoxy]-3-(4-phenylpiperazinyl-1)-propanol-2

Die erfindungsgemäßen Verbindungen der Formel I, ihre physiologisch verträglichen Salze und Säureadditionssalze und N-Oxide sind therapeutische Wirkstoffe, besitzen hohe pharmakologische Wirkung und sind wertvolle Arzneimittel. Insbesondere zeigen sie positiv inotrope, vasodilatierende, broncholytische sowie plättchenaggregationshemmende Wirkung und eignen sich zur Behandlung von Herzinsuffizienz, Hypertonie, Asthma und Thrombose.

Die Verbindungen der vorliegenden Erfindung können beim Menschen oral oder parenteral in einer Dosierung von 1 - 800 mg, vorzugsweise 10 - 200 mg, besonders bevorzugt 20 - 100 mg pro Tag, angewendet werden, insbesondere in unterteilten Dosen, zum Beispiel dreimal täglich. Diese Dosierungen sind vorteilhaft für die Behandlung der vorstehend genannten Krankheiten, insbesondere von Herzinsuffizienz und/oder Hypertonie.

Die positiv inotrope Wirkung der erfindungsgemäßen Verbindungen wurde am Meerschweinchen-Papillarmuskel bestimmt (Naunyn-Schmiedeberg's Arch. Pharmacol. 304, 37, 1978). Die Konzentration der Substanz im Organbad betrug jeweils $10^{-4}$mol/l. Die maximale prozentuale Steigerung der Kontraktionsamplitude wurde jeweils an drei Papillarmuskeln bestimmt und betrug mindestens 50%.

Gemäß der Erfindung werden pharmazeutische Zusammensetzungen geschaffen, die eine Verbindung der Formel I oder deren N-Oxide oder deren pharmazeutisch verträgliche Salze, zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten oder parenteral in Form von Lösungen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurfurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyoxyethylenstearat und Polyoxyethylensorbitanmonooleat und Konservierungsmitteln wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate wie Tabletten und Kapseln bevorzugt. Vorzugsweise enthalten die Präparate den Wirkstoff in einer Menge von 5 - 40 mg.

Die neuen Verbindungen der allgemeinen Formel I können hergestellt werden durch Umsetzung von

Verbindungen der Formel II,

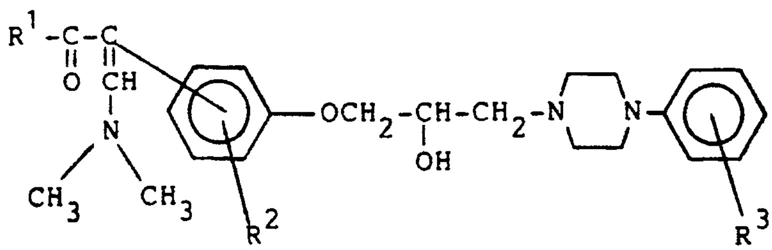

(II)

in der $R^1$, $R^2$ und $R^3$ die für Formel I angegebenen Bedeutungen haben, mit Cyanacetamid.

Die Reaktion der Verbindungen der Formel II mit Cyanacetamid erfolgt vorzugsweise durch Erhitzen in einem geeigneten Lösungsmittel, z.B. in einem niederen Alkohol wie Methanol oder Ethanol oder einem aprotischen Lösungsmittel wie Dimethylformamid, Tetrahydrofuran, Acetonitril, Diethylether oder Toluol in Gegenwart eines basischen Kondensationsmittels, vorzugsweise eines Alkalialkoholats, Alkalicarbonats oder Alkalihydrids. Als besonders geeignet hat sich eine Verwendung von Natriumcarbonat in Gegenwart eines Phasentransferkatalysators, vorzugsweise Tetrabutylammoniumhydrogensulfat, erwiesen. Bevorzugtes Lösungsmittel ist bei diesem Verfahren Acetonitril. Die Reaktionstemperaturen liegen zwischen Raumtemperatur und 150° C, vorzugsweise zwischen 50 und 100° C. Die Reaktionszeiten variieren zwischen 8 und 72 Stunden. Die optimalen Reaktionszeiten werden zweckmäßigerweise durch Dünnschichtchromatographie an Kieselgel bestimmt.

Die Verbindungen der Formel II, in der $R^1$, $R^2$ und $R^3$ die für Formel I angegebenen Bedeutungen haben, können hergestellt werden durch Umsetzung von Arylketonen der Formel III

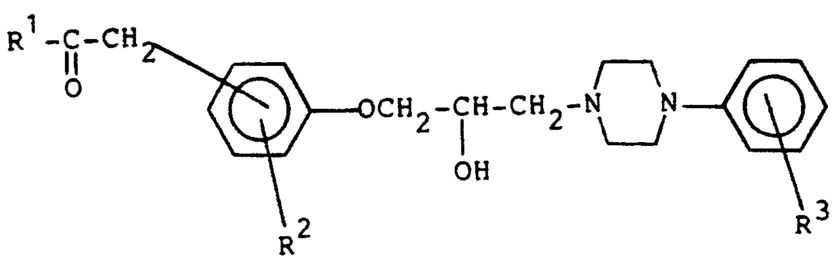

(III)

in der $R^1$, $R^2$ und $R^3$ die angegebene Bedeutung haben mit Dimethylformamiddimethylacetal. Die Reaktionen können in einem Lösungsmittel wie Dimethylformamid, Methanol oder Ethanol bei Temperaturen zwischen 50° C und der Siedetemperatur des Reaktionsgemisches, vorzugsweise zwischen 60 und 100° C, durchgeführt werden.

Arbeitet man ohne Lösungsmittel mit einem Überschuß an Dimethylformamiddimethylacetal, liegen die Reaktionszeiten zwischen einer und acht Stunden.

Die Verbindungen der Formel III können hergestellt werden aus Verbindungen der Formel IVa und IVb und deren Gemischen,

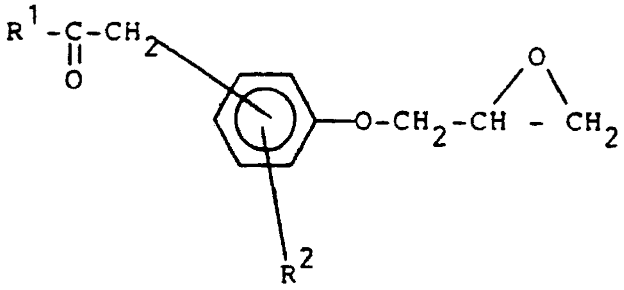

(IVa)

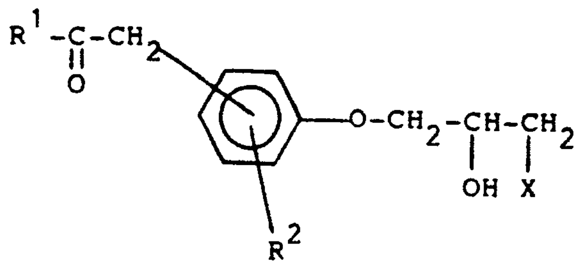

(IVb)

in der $R^1$ und $R^2$ die in Formel I angegebenen Bedeutungen haben und X Halogen, vorzugsweise Chlor oder Brom ist, mit einem Piperazinderivat der Formel V,

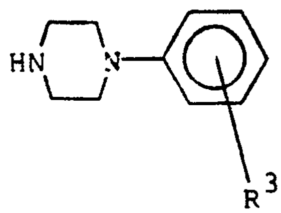

(V)

in der $R^3$ die in Formel I angegebenen Bedeutungen hat. Die Umsetzungen werden vorzugsweise in einem Lösungsmittel bei Temperaturen zwischen 20 und 120 °C, zweckmäßig bei 50 - 120 °C durchgeführt.

Als Lösungsmittel werden vorzugsweise niedere Alkohole mit 1 bis 4 Kohlenstoffatomen, insbesondere Ethanol oder Isopropanol, verwendet. Die Umsetzungen können jedoch auch in Ethern wie Diethylether, Tetrahydrofuran oder Dioxan, Dimethylformamid oder Dimethylsulfoxid durchgeführt werden. Die Reaktionsdauer hängt von der Reaktionstemperatur ab und beträgt im allgemeinen 2 bis 15 Stunden.

Die Verbindungen der Formel IV, in der $R^1$ und $R^2$ die in Formel I angegebenen Bedeutungen haben, können hergestellt werden durch Alkylierung von Phenolen der allgemeinen Formel VI,

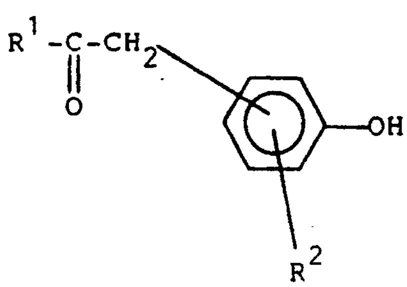

(VI)

in der $R^1$ und $R^2$ die für Formel I angegebenen Bedeutungen haben, mit einem Epihalogenhydrin wie Epichlorhydrin oder Epibromhydrin.

Die Alkylierungen der Phenolderivate der Formel VI werden zweckmäßigerweise bei Temperaturen von 0 - 120 °C in einem inerten Lösungsmittel wie Aceton, einem niederen Alkohol mit 1 bis 4 Kohlenstoffatomen wie Methanol, Ethanol, Propanol oder Butanol, einem Ether wie Diethylether, Tetrahydrofuran oder Dioxan oder Dimethylformamid, Dimethylsulfoxid oder in überschüssigem Alkylierungsmittel als Lösungsmittel durchgeführt. Bevorzugt werden die Umsetzungen in Gegenwart einer Base als säurebindendes Mittel durchgeführt. Geeignete Basen sind Alkalicarbonate, -hydrogencarbonate, -hydride oder -hydroxide, insbesondere des Natriums und Kaliums. Bevorzugt werden die Phenol-Derivate mit Epichlorhydrin in Dimethylsulfoxid in Gegenwart von Natronlauge bei Raumtemperatur oder Temperaturen bis 50 °C umgesetzt.

Die verwendeten Ausgangsmaterialien sind bekannt oder können nach an sich bekannten Verfahren bzw. analog zu den hier beschriebenen oder analog zu an sich bekannten Verfahren hergestellt werden.

Die Verbindungen der allgemeinen Formel I können sowohl Basen als auch Säuren, beziehungsweise amphoter sein und daher in der Form ihrer Salze oder Säureadditionssalze aus den Reaktionsgemischen isoliert werden. Sie lassen sich als Basen mit geeigneten anorganischen oder organischen Säuren nach bekannten Verfahren in Salze überführen oder bilden als Säuren mit Basen Salze.

Bevorzugt werden physiologisch verträgliche Salze oder Säureadditionssalze. Hierfür sind als anorganische Säuren beispielsweise Halogenwasserstoffsäuren, zum Beispiel Salzsäure oder Schwefelsäure, und als organische Säuren zum Beispiel Fumarsäure, Maleinsäure, Zitronensäure und Weinsäure geeignet. Zur Herstellung wird die heiße alkoholische Lösung der Base mit der alkoholischen Lösung einer geeigneten Säure versetzt und man erhält nach Etherzusatz das Salz. Bevorzugte Salze sind die Alkali-, Erdalkali- und Ammoniumsalze der Verbindungen der Formel I, die mit den entsprechenden Basen, insbesondere Natrium-, Kalium- oder Ammoniumhydroxid erhalten werden.

Die erfindungsgemäßen Verbindungen der Formel I weisen am Kohlenstoffatom 2 der Isopropanol-Kette ein Chiralitätszentrum auf und können, abhängig von den Substituenten, weitere asymmetrische Kohlenstoffatome besitzen und daher als Racemate und Diastereoisomere vorliegen. Diastereoisomere können in bekannter Weise aufgrund der physikalisch-chemischen Unterschiede ihrer Bestandteile in ihre racemischen Modifikationen getrennt werden. Racemate können nach bekannten Methoden getrennt werden, beispielsweise durch Umkristallisieren in optisch aktiven Lösungsmitteln, durch Mikroorganismen oder Reaktion mit einer optisch aktiven Säure oder Base, die mit der racemischen Verbindung ein Salz bildet, Trennung der Diastereoisomeren durch fraktionierte Kristallisation und Freisetzung der Enantiomeren durch geeignete Mittel. Besonders geeignete optisch aktive Säuren sind beispielsweise die d- und 1-Formen der Weinsäure, Ditoluylweinsäure, Äpfelsäure, Mandelsäure, Kamphersulfonsäure oder Pyrrolidon-carbonsäure. Geeignete optisch aktive Basen sind α-Phenyläthylamin, Menthylamin, Ephedrin, Brucin und Chinin. Vorteilhafterweise wird der aktivere der Antipoden isoliert. Gemäß der Erfindung ist es jedoch auch möglich, die reinen Enantiomeren durch asymmetrische Synthese zu erhalten.

Die folgenden Beispiele erläutern die Erfindung:

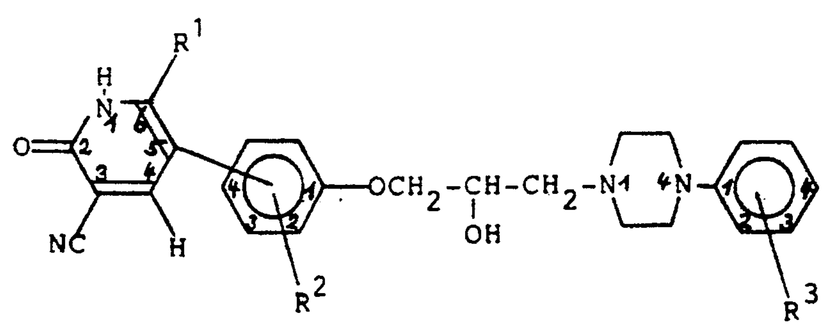

(I)

Beispiel 1

1-[4-(3-Cyano-1,2-dihydro-6-methyl-2-oxo-pyridinyl-5)-phenoxy]-3-[4-(2-methoxyphenyl)-piperazinyl-1]-propanol-2

a) 32,8 4-Hydroxyphenylaceton werden unter Kühlung in 175g 5%iger Natronlauge gelöst und mit 60 ml DMSO versetzt. Danach werden bei Temperaturen unterhalb 20 °C 35 ml Epichlorhydrin zugetropft. Man läßt den Ansatz über Nacht stehen, extrahiert mit Chloroform, wäscht den Extrakt mit Wasser, trocknet

mit Calciumchlorid und entfernt im Wasserstrahlpumpenvakuum das Lösungsmittel. Der Rückstand wird bei 0.2 mbar an der Ölpumpe destilliert.

Kp.: 139-141 °C. Man erhält 33,2 g 4-(2,3-Epoxypropoxy)-phenylaceton
Schmp.: 43 °C

b) 30,0 g 4-(2,3-Epoxypropoxy)-phenylaceton und 25,6 g 2-Methoxyphenyl-piperazin werden in 300 ml Ethanol 3 Stunden unter Rückfluß erhitzt. Danach wird das Lösungsmittel abdestilliert. Der Rückstand wird mit Essigester/Hexan zur Kristallisation gebracht. Man erhält 32,5 g 1-[4-(Propan-2-on-yl)-phenoxy]-3-[4-(2-methoxyphenyl)-piperazinyl-1]-propanol-2.
Schmp. 42-43 °C.

c) 32,0 g 1-[4-Propan-2-on-yl)-phenoxy]-3-[4-(2-methoxyphenyl)-piperazinyl-1]-propanol-2 werden in 100 ml Methanol gelöst, mit 20,0 ml Dimethylformamiddimethylacetal versetzt und über Nacht unter Rückfluß erhitzt. Danach wird das Lösungsmittel im Vakuum abdestilliert, der Rückstand mit Petrolether verrieben und mit Ether bei -60 °C zur Kristallisation gebracht. Man erhält 28,5 g 1-[4-(4-Dimethylamino-3-buten-2-on-3-yl)-phenoxy]-4-[4-(2-methoxyphenyl)-piperazinyl-1]propanol-2.
Schmp. 185 °C

d) 9,0 g 1-[4-(4-Dimethylamino-3-buten-2-on-3-yl)-phenoxy]-3-[4-(2-methoxyphenyl)-piperazinyl-1]-propanol-2 werden in 100 ml Acetonitril mit 5,66 g Kaliumcarbonat, 0,67g Tetrabutyl-ammoniumhydrogensulfat und 2,52 g Cyanacetamid 36 Stunden unter Rückfluß erhitzt. Danach wird das Lösungsmittel im Vakuum abdestilliert, der Rückstand in 50 ml Wasser gelöst, mit verdünnter Salzsäure neutralisiert und mehrmals mit Essigester extrahiert. Die Extrakte werden getrocknet und eingeengt. Der Rückstand wird säulenchromatographisch an Kieselgel gereinigt (Chloroform : Methanol = 10 : 1).
Man erhält 2,2 g 1-[4-(3-Cyano-1,2-dihydro-6-methyl-2-oxo-pyridinyl-5)-phenoxy]-3-[4-(2-methoxyphenyl)-piperazinyl-1]-propanol-2.
Schmp. 159-160 °C

e) 2,0 g dieses Produkts werden in 20 ml absolutem Methanol suspendiert. Man tropft unter Kühlung etherische HCl zu, bis pH 3 erreicht ist und versetzt dann mit 200 ml Ether, kühlt einige Stunden auf 0 °C und saugt den Niederschlag ab. Man erhält 2,1 g 1-[4-(3-Cyano-1,2-dihydro-6-methyl-2-oxo-pyridinyl-5)-phenoxy]-3-[4-(2-methoxyphenyl)-piperazinyl-1]-propanol-2-hydrochlorid.
Schmp. 298 °C

Beispiel 2

1-[4-(3-Cyano-1,2-dihydro-6-methyl-2-oxo-pyridinyl-5)-phenoxy]-3-[4-phenyl-piperazinyl-1]-propanol-2

a) 10,0 g 4-(2,3-Epoxypropoxy)phenylaceton werden in 60 ml Ethanol gelöst und mit 7,64 g Phenylpiperazin 3 Stunden unter Rückfluß erhitzt. Man läßt abkühlen und saugt das auskristallisierte Produkt ab. Man erhält 16,05 g 1-[4-(Propan-2-on-yl)-phenoxy]-3-(4-phenylpiperazinyl-1)-propanol-2.
Schmp. 121-122 °C

b) 14,0 g 1-[4-(Propan-2-on-yl)-phenoxy]-3-(4-phenylpiperazinyl-1)-propanol-2 und 18 ml Dimethylformamiddimethylacetal werden in 60 ml Methanol gelöst und 5 Stunden unter Rückfluß erhitzt. Man läßt abkühlen, saugt das Produkt ab und verreibt den Rückstand mit Ether. Man erhält 13,1g 1-[4-(4-Dimethylamino-3-buten-2-on-3-yl)-phenoxy]-4-(4-phenylpiperazinyl-1)-propanol-2.
Schmp. 153-154 °C

c) Eine Lösung von 3,0 g Natriumethylat in 130 ml Ethanol wird mit 1,93 g Cyanacetamid und 10,0g 1-[4-(4-Dimethylamino-3-buten-2-on-3-yl-phenoxy]-3-(phenylpiperazinyl-1)-propanol-2 versetzt und 40 Stunden unter Ruckfluß erhitzt. Man läßt abkühlen, saugt die Kristalle ab und wäscht mit Wasser und Ethanol. Man fällt das Hydrochlorid analog Beispiel 1e) und erhält 5,95 g 1-[4-(3-Cyano-1,2-dihydro-6-methyl-2-oxo-pyridinyl-5-)-phenoxy]-3-[4-phenyl-piperazinyl-1]-propanol-2-hydrochlorid-hydrat
Schmp. 206-207 °C

Beispiel 3

1-[4-(3-Cyano-1,2-dihydro-6-methyl-2-oxo-pyridinyl-5)-phenoxy]-3-[4-(2-methylphenyl)-piperazinyl-1]-propanol-2

a) 4-(2,3-Epoxypropoxy)-phenylaceton wird analog Beispiel 2a) mit 2-Methylphenyl-piperazin umgesetzt. Man erhält 1-[4-(Propan-2-on-yl)-phenoxy]-3-[4-(2-methylphenyl)-piperazinyl-1]-propanol-2.
Schmp. 232 °C

b) Dieses Produkt wird analog Beispiel 2b) mit Dimethylformamiddimethylacetal umgesetzt. Man erhält 1-[4-(Dimethylamino-3-buten-2-on-3-yl)-phenoxy]-3-[4-(2-methylphenyl)-piperazinyl-1]-propanol-2.
Schmp. 234-236° C

c) Die Umsetzung dieser Substanz mit Cyanacetamid analog 1d) und die Überführung des Reaktionsprodukts ins Hydrochlorid analog 1e) liefert 1-[4-(3-Cyano-1,2-dihydro-6-methyl-2-oxo-pyridinyl-5)-phenoxy]-3-[4-(2-methylphenyl)-piperazinyl-1]-propanol-2-hydrochlorid.
Schmp. 292° C

Beispiel 4

1-[2-Methoxy-4-(3-cyano-1,2-dihydro-6-methyl-2-oxo-pyridinyl-5)-phenoxy]-3-[4-(2-methoxyphenyl)-piperazinyl-1]-propanol-2

a) 4-(2,3-Epoxypropxy)-2-methoxyphenylaceton wird analog Beispiel 2a) mit 2-Methoxyphenyl-piperazin umgesetzt. Man erhält 1-[2-Methoxy-4-(propan-2-on-yl)-phenoxy]-3-[4-(2-methylphenyl)-piperazinyl-1]-propanol-2.
Schmp. 128° C

b) Nach Umsetzung dieser Verbindung mit Dimethylformamiddimethylacetal analog 2b) erhält man 1-[4-(4-Dimethylamino-3-buten-2-on-3-yl)-2-methoxy-phenoxy]-3-[4-(2-methylphenyl)-piperazinyl-1]-propanol-2.
Schmp. 95° C

c) Aus dieser Verbindung erhält man durch Reaktion mit Cyanacetamid analog 1d) und anschließende Hydrochloridfällung analog 1e) 1-[2-Methoxy-4-(3-cyano-1,2-dihydro-6-methyl-2-oxo-pyridinyl-5)-phenoxy]-3-[4-(2-methoxyphenyl)-piperazinyl-1]-propanol-2-di-hydrochlorid.
Schmp. 176-178° C

Beispiel 5

1-[3-(3-Cyano-1,2-dihydro-6-methyl-2-oxo-pyridinyl-5)-phenoxy]-3-[4-(2-methoxyphenyl)-piperazinyl-1]-propanol-2

a) 3-(2,3-Epoxypropoxy)-phenylaceton wird analog Beispiel 2a) mit 2-Methoxyphenylpiperazin umgesetzt. Man erhält 1-[3-(Propan-2-on-yl)-phenoxy]-3-[4-(2-methoxyphenyl)-piperazinyl-1]-propanol-2 als sirupöses Produkt.

b) Die Umsetzung dieser Verbindung mit Dimethylformamiddimethylacetal analog Beispiel 2b) liefert 1-[3-(4-Dimethylamino-3-buten-2-on-3-yl)-phenoxy]-3-[4-(2-methoxyphenyl)-piperazinyl-1]-propanol-2 als sirupöses Produkt.

c) Aus dieser Verbindung erhält man durch Reaktion mit Cyanacetamid analog Beispiel 1d) und Hydrochloridfällung analog Beispiel 1c) 1-[3-(3-Cyano-1,2-dihydro-6-methyl-2-oxo-pyridinyl-5)-phenoxy]-3-[4-(2-methoxyphenyl)-piperazinyl-1]-propanol-2-dihydrochlorid.
Schmp. 174-175° C

Analog zu den vorstehenden Beispielen werden die in der folgenden Tabelle angegebenen erfindungsgemäßen Verbindungen der Formel I erhalten, in der der Pyridinonring in 4-Position steht:

| Beispiel | $R^1$ | $R^2$ | $R^3$ | Salz | Schmp.°C |
|---|---|---|---|---|---|
| 6 | $CH_3$ | H | 3-$OCH_3$ | di-Hydrochlorid-Hydrat | 239 |
| 7 | $CH_3$ | H | 4-OH | Hydrochlorid | 262 |
| 8 | $CH_3$ | H | 2-F | Hydrochlorid | 253-254 |
| 9 | $CH_3$ | H | 2-Cl | Hydrochlorid-Hydrat | 298-299 |
| 10 | $CH_3$ | 3-$OCH_3$ | 2-$OCH_3$ | Hydrochlorid | 234 |
| 11 | $CH_3$ | H | 2-$CF_3$ | di-Hydrochlorid-Hydrat | 246-247 |

| Beispiel | $R^1$ | $R^2$ | $R^3$ | Salz | Schmp.°C |
|---|---|---|---|---|---|
| 12 | $CH_3$ | H | 2-$NO_2$ | Hydrochlorid | 259 |
| 13 | $CH_3$ | H | 4-$NO_2$ | Hydrochlorid-Hydrat | 258-259 |
| 14 | $CH_3$ | H | 2-$OC_2H_5$ | Hydrochlorid-Hydrat | 230 |
| 15 | $CH_3$ | H | 3-$CF_3$ | Dihydrochlorid-Hydrat | 246-247 |
| 16 | $CH_3$ | H | 3-Cl | Hydrochlorid | 284-285 |
| 17 | $CH_3$ | H | 4-F | Hydrochlorid-Hydrat | 251-252 |
| 18 | $CH_3$ | H | 2-$C_2H_5$ | Hydrochlorid-Hydrat | 263-264 |
| 19 | $CH_3$ | H | 2-OH | Hydrochlorid-Hydrat | 283-284 |
| 20 | $CH_3$ | H | 2-Br | Hydrochlorid | 298 (Z) |
| 21 | $CH_3$ | H | 2-CN | Hydrochlorid | 157-158 |
| 22 | $C_2H_5$ | H | 2-$OCH_3$ | Hydrochlorid | 250 |
| 23 | $CH_3$ | H | 4-Cl | Hydrochlorid | 232 |

Beispiel 24

Herstellung von Tabletten und Kapseln

Tabletten und Kapseln, welche die nachstehend angegebenen Bestandteile enthalten, werden nach bekannten Arbeitsweisen hergestellt. Diese sind für die Behandlung der vorstehend genannten Krankheiten, insbesondere der Herzinsuffizienz in Dosierungsmengen von jeweils einer Tablette oder Kapsel dreimal täglich geeignet.

| Bestandteile | Gewicht (mg) Tablette | Kapsel |
|---|---|---|
| 1-/4-(3-Cyano-1,2-dihydro-6-methyl-2-oxo-pyridinyl-5)-phenoxy7-3-/4-(2-methoxy-phenyl)-piperazinyl-17-propanol-2-hydrochlorid | 10 | 10 |
| Tragacanth | 10 | - |
| Lactose | 247,5 | 300 |
| Maisstärke | 25 | - |
| Talk | 15 | - |
| Magnesiumstearat | 2,5 | - |

Beispiel 25

Herstellung von Ampullen

Ampullen, die die im folgenden genannten Bestandteile enthalten, lassen sich in bekannter Weise herstellen. Wirkstoff und Natriumchlorid werden in Wasser gelöst und unter Stickstoff in Glasampullen abgefüllt.

| | |
|---|---|
| 1-/4-(3-Cyano-1,2-dihydro-6-methyl-2-oxo-pyridinyl-5)-phenoxy7-3-/4-(2-methoxyphenyl)-piperazinyl-17-propanol-2-hydrochlorid | 2 mg |
| Natriumchlorid | 18 mg |
| dest. Wasser ad | 2,0 ml |

## Patentansprüche

**1.** Substituierte Phenylpiperazinyl-propanole der Formel I,

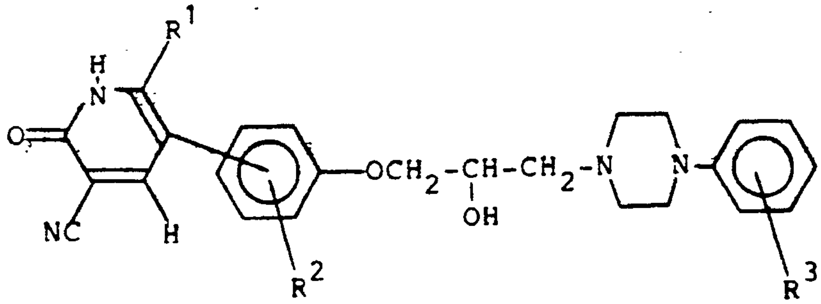

(I)

in der

R[1] Wasserstoff, eine Alkylgruppe mit 1 bis 4 Kohlenstoffatomen und

R[2] und R[3], die gleich oder verschieden sein können, jeweils Wasserstoff, Halogen, Cyano, eine Trifluormethylgruppe, eine Nitrogruppe, eine Hydroxygruppe, eine Alkoxygruppe oder eine Alkylgruppe mit jeweils 1 bis 4 Kohlenstoffatomen bedeuten, wobei die Alkylteile jeweils geradkettig oder verzweigt sein können, sowie ihre tautomeren Formen und ihre Salze sowie Säureadditionssalze und N-Oxide.

**2.** 1-[4-(3-Cyano-1,2-dihydro-6-methyl-2-oxo-pyridinyl-5)-phenoxy]-3-[4-(2-methoxyphenyl)-piperazinyl-1]-propanol-2 nach Anspruch 1.

**3.** 1-[4-(3-Cyano-1,2-dihydro-6-methyl-2-oxo-pyridinyl-5)-phenoxy]-3-[4-(2-methylphenyl)-piperazinyl-1]-propanol-2 nach Anspruch 1.

**4.** 1-[4-(3-Cyano-1,2-dihydro-6-methyl-2-oxo-pyridinyl-5)-2-methoxyphenoxy]-3-[4-(2-methoxyphenyl)-piperazinyl-1]-propanol-2 nach Anspruch 1.

**5.** 1-[4-(3-Cyano-1,2-dihydro-6-methyl-2-oxo-pyridinyl-5)-phenoxy]-3-(4-phenylpiperazinyl-1)-propanol-2 nach Anspruch 1.

**6.** Verfahren zur Herstellung der Verbindungen der Formel I gemäß Anspruch 1, dadurch gekennzeichnet, daß man Verbindungen der Formel II

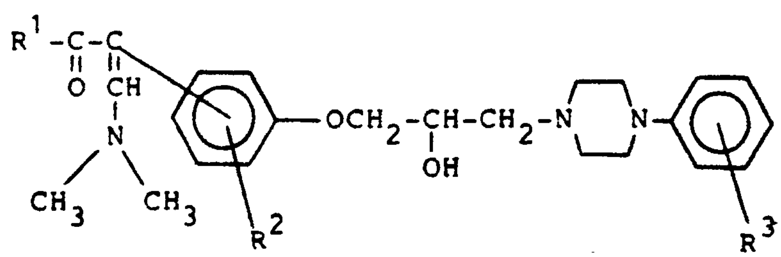

(II)

in der R[1], R[2] und R[3] die angegebenen Bedeutungen haben, mit Cyanacetamid umsetzt.

**7.** Verfahren zur Herstellung der Verbindungen der Formel II, dadurch gekennzeichnet, daß man Verbindungen der Formel III

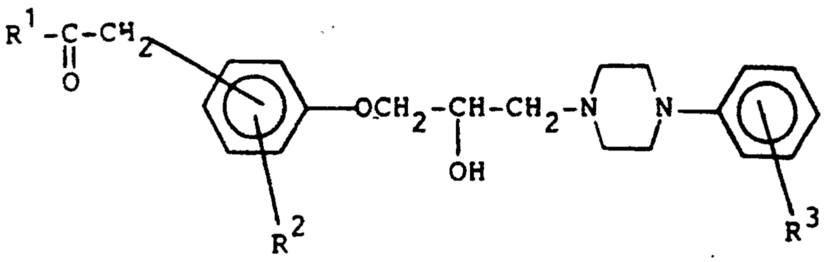

(III)

in der $R^1$, $R^2$ und $R^3$ die angegebene Bedeutung haben, mit Dimethylformamiddimethylacetal umsetzt.

8. Verfahren zur Herstellung der Verbindungen der allgemeinen Formel III, dadurch gekennzeichnet, daß man Verbindungen der Formeln IVa und IVb

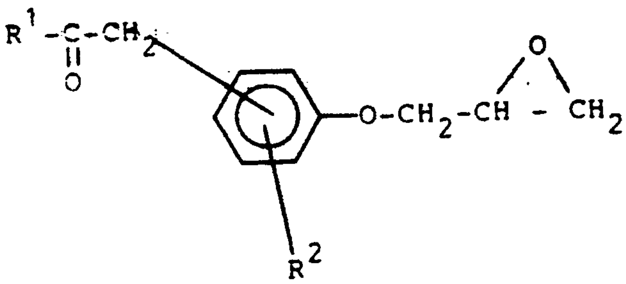

(IVa)

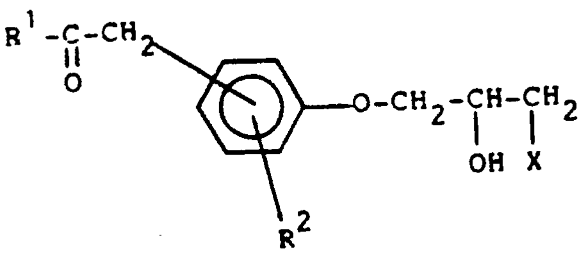

(IVb)

in der $R^1$, $R^2$ und X die angegebene Bedeutung haben, mit einem Piperazinderivat der Formel V

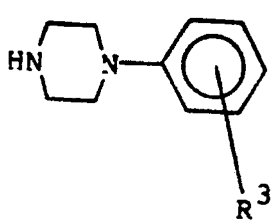

(V)

in der $R^3$ die angegebene Bedeutung hat, umsetzt.

9. Verfahren zur Herstellung der Verbindungen der Formel IVa und IVb, dadurch gekennzeichnet, daß man Verbindungen der Formel VI

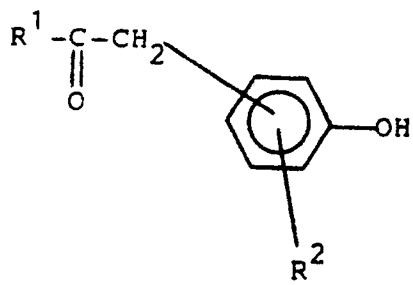

(VI)

in der $R^1$ und $R^2$ die angegebene Bedeutung haben, mit einem Epihalogenhydrin umsetzt.

**10.** Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

**11.** Verbindungen der Formel I gemäß Anspruch 1 zur Anwendung als therapeutische Wirkstoffe, insbesondere zur Anwendung bei Herzinsuffizienz, Hypertonie, Asthma und Thrombose.

**Claims**

**1.** Substituted phenylpiperazinylpropanols of the formula I

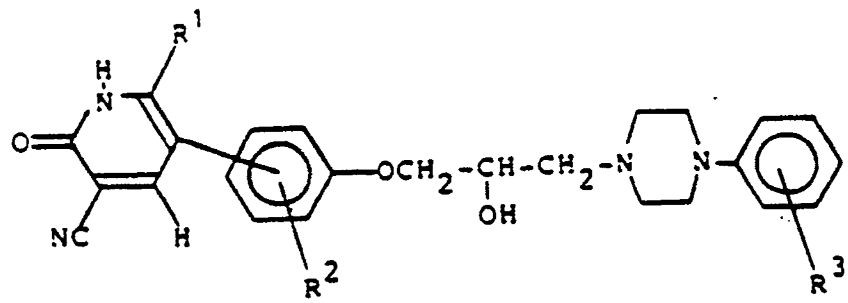

(I)

in which

$R^1$ denotes hydrogen or an alkyl group with 1 to 4 carbon atoms and

$R^2$ and $R^3$, which can be identical or different, each denote hydrogen, halogen, cyano, a trifluoromethyl group, a nitro group, a hydroxyl group, an alkoxy group or an alkyl group with in each case 1 to 4 carbon atoms, it being possible for the alkyl parts in each case to be straight-chain or branched, and their tautomeric forms and their salts as well as acid addition salts and N-oxides.

**2.** 1-[4-(3-Cyano-1,2-dihydro-6-methyl-2-oxopyridin-5-yl)phenoxy]-3-[4-(2-methoxyphenyl)piperazin-1-yl]-propan-2-ol according to Claim 1.

**3.** 1-[4-(3-Cyano-1,2-dihydro-6-methyl-2-oxopyridin-5-yl)phenoxy]-3-[4-(2-methylphenyl)piperazin-1-yl]-propan-2-ol according to Claim 1.

**4.** 1-[4-(3-Cyano-1,2-dihydro-6-methyl-2-oxopyridin-5-yl)-2-methoxyphenoxy]-3-[4-(2-methoxyphenyl)-piperazin-1-yl]propan-2-ol according to Claim 1.

**5.** 1-[4-(3-Cyano-1,2-dihydro-6-methyl-2-oxopyridin-5-yl)phenoxy]-3-(4-phenylpiperazin-1-yl)propan-2-ol according to Claim 1.

**6.** Process for the preparation of the compounds of the formula I according to Claim 1, characterised in that compounds of the formula II

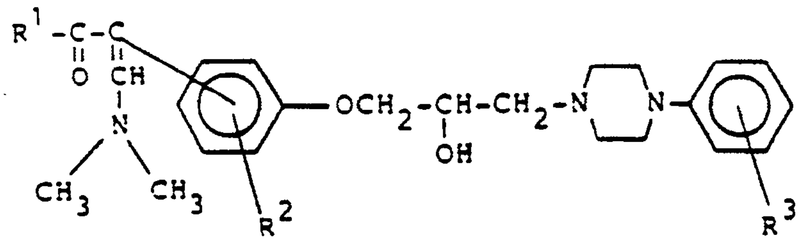

(II)

in which $R^1$, $R^2$ and $R^3$ have the meanings given, are reacted with cyanoacetamide.

7. Process for the preparation of the compounds of the formula II, characterised in that compounds of the formula III

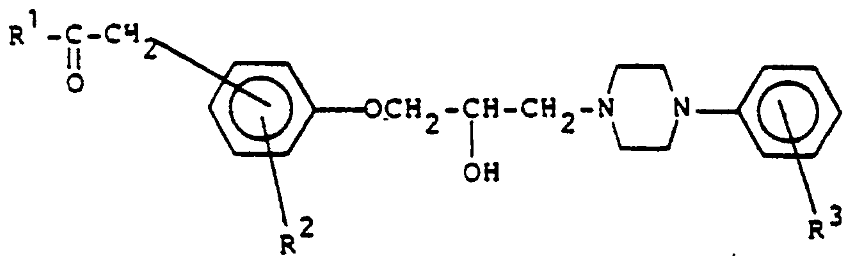

(III)

in which $R^1$, $R^2$ and $R^3$ have the meaning given, are reacted with dimethylformamide dimethyl acetal.

8. Process for the preparation of the compounds of the general formula III, characterised in that compounds of the formulae IVa and IVb

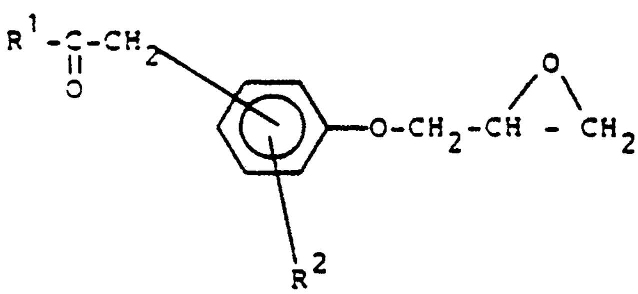

(IVa)

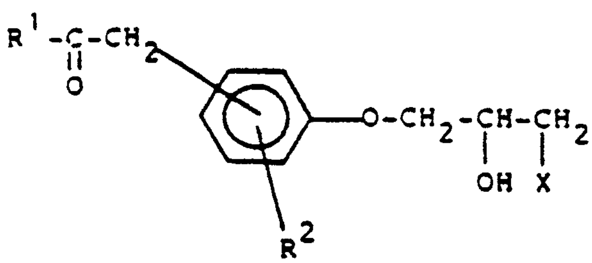

(IVb)

in which $R^1$, $R^2$ and X have the meaning given, are reacted with a piperazine derivative of the formula V

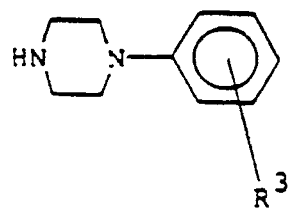

(V)

in which $R^3$ has the meaning given.

9. Process for the preparation of the compounds of the formula IVa and IVb, characterised in that compounds of the formula VI

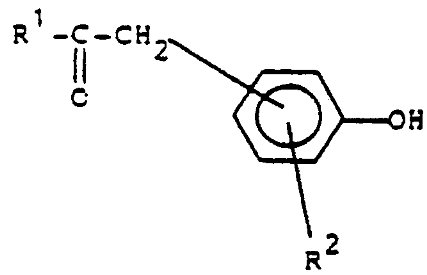

(VI)

in which $R^1$ and $R^2$ have the meaning given, are reacted with an epihalohydrin.

10. Pharmaceutical product, characterised in that it contains one or more of the compounds according to Claim 1 or physiologically acceptable salts thereof and, if appropriate, customary excipients and/or diluents.

11. Compounds of the formula I according to Claim 1 for use as therapeutic active compounds, in particular for use in cases of cardiac insufficiency, hypertension, asthma and thrombosis.

## Revendications

1. Phénylpipérazinylpropanols substitués de formule I

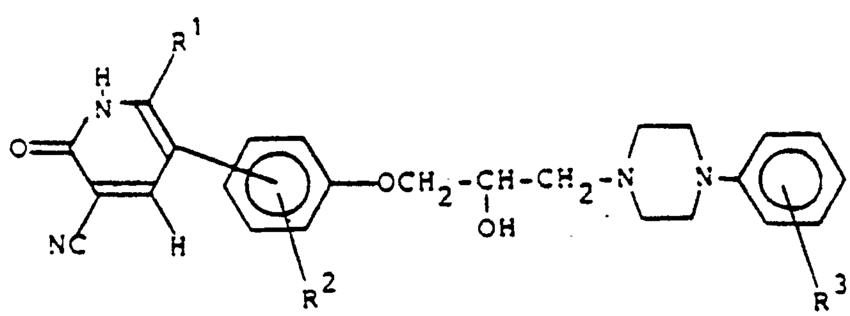

(I)

dans laquelle

| | |
|---|---|
| $R^1$ | désigne un hydrogène ou un groupement alcoyle ayant de 1 à 4 atomes de carbone et |
| $R^2$ et $R^3$, | pouvant être identiques ou différents, désignent chacun un hydrogène, un halogène, un cyano ou un groupement trifluorométhyle, un groupement nitro, un groupement hydroxyle, un groupement alcoxy ou un groupement alcoyle, ayant dans chaque cas de 1 à 4 atomes de carbone, les motifs alcoyles pouvant être dans chaque cas une chaîne linéaire ou ramifiée, et leur formes tautomères et leur sels ainsi que des sels d'addition acides et des N-oxydes. |

**2.** 1-[4-(3-Cyano-1,2-dihydro-6-méthyl-2-oxopyridin-5-yl)phénoxy]-3-[4-(2-méthoxyphényl)pipérazin-1-yl] propan-2-ol selon la revendication 1.

**3.** 1-[4-(3-Cyano-1,2-dihydro-6-méthyl-2-oxopyridin-5-yl)phénoxy]-3-[4-(2-méthylphényl)pipérazin-1-yl]-propan-2-ol selon la revendication 1.

**4.** 1-[4-(3-Cyano-1,2-dihydro-6-méthyl-2-oxopyridin-5-yl)-2-méthoxyphénoxy]-3-[4-(2-méthoxyphényl)-pipérazin-1-yl]propan-2-ol selon la revendication 1.

**5.** 1-[4-(3-Cyano-1,2-dihydro-6-méthyl-2-oxopyridin-5-yl)phénoxy]-3(4-phénylpipérazin-1-yl)propan-2-ol selon la revendication 1.

**6.** Procédé de préparation des composés de formule I selon la revendication 1, caractérisé en ce que l'on fait réagir des composés de formule II

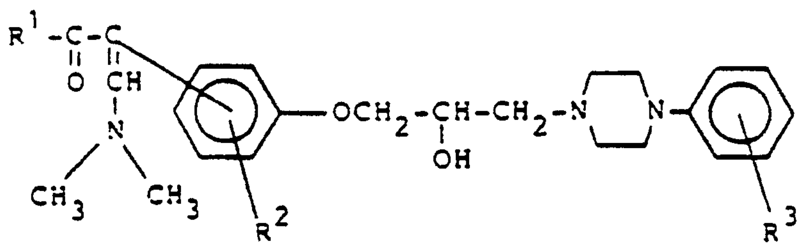

(II)

dans laquelle $R^1$, $R^2$ et $R^3$ sont comme définis, avec du cyanoacétamide.

**7.** Procédé de préparation des composés de formule II, caractérisé en ce que l'on fait réagir des composés de formule III

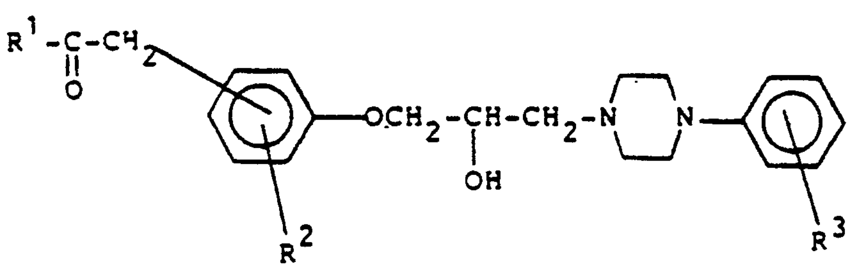

(III)

dans laquelle $R^1$, $R^2$ et $R^3$ sont comme définis, avec du diméthylformamide-diméthylacétal.

**8.** Procédé de préparation des composés de formule générale III, caractérisé en ce que l'on fait réagir des composés de formules IVa et IVb

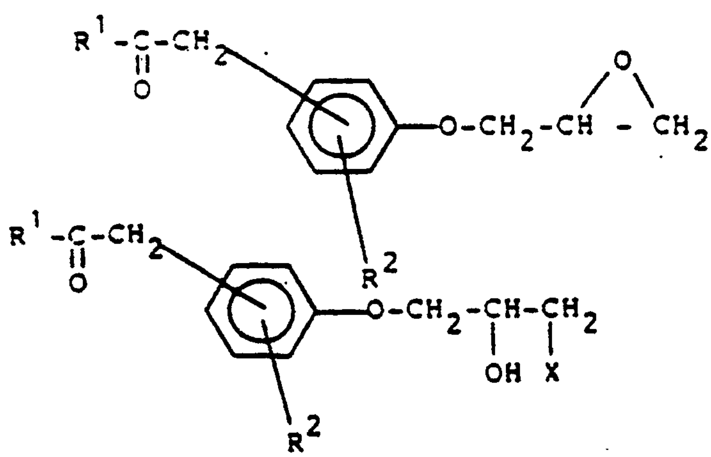

(IVa)

(IVb)

dans laquelle $R^1$, $R^2$ et X sont comme définis, avec un dérivé de pipérazine de formule V

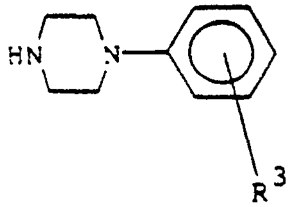

(V)

dans laquelle $R^3$ est comme défini.

9. Procédé de préparation des composés de formule IVa et IVb, caractérisé en ce que l'on fait réagir des composés de formule VI

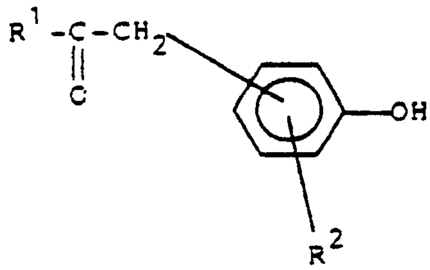

(VI)

dans laquelle $R^1$, et $R^2$ sont comme définis, avec une épihalohydrine.

10. Produit pharmaceutique caractérisé en ce qu'il contient un ou plusieurs des composés selon la revendication 1, ou des sels physiologiquement acceptables de ces derniers et éventuellement des excipients et/ou des diluants habituels.

11. Composés de formule I selon la revendication 1, pour utilisation à titre de composés thérapeutiques actifs, en particulier pour utilisation dans des cas d'insuffisance cardiaque, d'hypertension d'asthme et de thrombose.